(19) **Europäisches Patentamt**

**European Patent Office**

**Office européen des brevets**

(11) **EP 1 303 745 B1**

(12) **EUROPÄISCHE PATENTSCHRIFT**

(45) Veröffentlichungstag und Bekanntmachung des Hinweises auf die Patenterteilung:
**21.06.2006 Patentblatt 2006/25**

(21) Anmeldenummer: **01909658.5**

(22) Anmeldetag: **19.01.2001**

(51) Int Cl.:
*G01N 9/00* (2006.01)    *A61B 8/00* (2006.01)
*G01N 29/024* (2006.01)    *G01N 29/07* (2006.01)

(86) Internationale Anmeldenummer:
**PCT/EP2001/000579**

(87) Internationale Veröffentlichungsnummer:
**WO 2002/010711 (07.02.2002 Gazette 2002/06)**

(54) **VORRICHTUNG ZUR FESTSTELLUNG DER ÄNDERUNG DER DICHTE EINES MEDIUMS**

DEVICE FOR DETERMINING THE CHANGE IN THE DENSITY OF A MEDIUM

APPAREIL PERMETTANT DE CONSTATER LA MODIFICATION DE DENSITE D'UN FLUIDE

(84) Benannte Vertragsstaaten:
**AT BE CH CY DE DK ES FI FR GB GR IE IT LI LU MC NL PT SE TR**

(30) Priorität: **27.07.2000 DE 10036565**

(43) Veröffentlichungstag der Anmeldung:
**23.04.2003 Patentblatt 2003/17**

(73) Patentinhaber: **Visit Video-Stroboskop Innovations-Technik GmbH & Co. KG**
**97076 Würzburg (DE)**

(72) Erfinder:
 • **GLASER, Eckard,**
 **c/o Soem GMBH**
 **97076 Würzburg (DE)**
 • **WROBEL, Miroslaw,**
 **c/o Sonem GMBH**
 **97082 Würzburg (DE)**

 • **GRASSMANN, Janet,**
 **c/o Sonem GMBH**
 **95246 Eibelstadt (DE)**

(74) Vertreter: **Blaumeier, Jörg**
 **Lindner, Blaumeier & Kollegen**
 **Patent- und Rechtsanwälte**
 **Postfach 119109**
 **90101 Nürnberg (DE)**

(56) Entgegenhaltungen:
**EP-A- 0 430 859**    **EP-A- 0 701 118**
**GB-A- 798 323**    **US-A- 4 059 010**
**US-A- 4 481 820**    **US-A- 5 804 698**
**US-A- 6 012 324**

 • **GEORG L. TRIGG: "Encyclopedia of Applied Physics" 1998 , WILEY-VCH VERLAG GMBH , WEINHEIM, GERMANY XP002167976 * Vol. 22, Chapter "Ultrasonic Biophysics" by L.A. Frizzell, p. 475-494 ***

EP 1 303 745 B1

**Beschreibung**

Die Erfindung betrifft eine Vorrichtung zur Feststellung der Änderung der Dichte eines festen, flüssigen oder gasförmigen Mediums. Die Vorrichtung ist insbesondere in der Lage, Einwirkungen von physikalischen und/oder chemischen Parametern, die Änderungen in der Dichte und/oder der Kompressionskonstanten des Mediums zur Folge haben, wie sie zum Beispiel bei Temperatur- oder Druckänderungen, bei einer chemischen, biochemischen oder physikalischen Reaktion auftreten, in ihrer Auswirkung auf die Dichte des Mediums zu erfassen.

Änderungen der Temperatur und des Druckes werden bekanntlich mit herkömmlichen Mitteln zur Temperatur- und Druckmessung erfasst. Diese Mittel versagen jedoch, wenn ein Medium schwer oder nicht zugänglich ist oder sich das Medium in einer Umgebung befindet, in der keine Messeinrichtungen eingebracht werden können. Außerdem sind diese Änderungen häufig so gering, dass sie nur mit sehr aufwendigen Messeinrichtungen gemessen werden können.

Bei vielen Vorgängen ist für den Anwender die Änderung von Temperatur und/oder Druck nur ein Hinweis dafür, dass das Medium eine bestimmte gewünschte Eigenschaft erreicht hat, z.B. dass ein Öl die erforderliche Viskosität besitzt oder dass ein empfindliches Gefriergut aufgetaut ist. Ein chemischer, biochemischer oder ein physikalischer Prozess muss nicht immer mit einer Temperatur- oder Druckänderung verbunden sein. Für den Nachweis, dass ein derartiger Prozess stattfindet oder stattgefunden hat, kann deshalb eine Temperatur- und/oder Druckmessung nicht herangezogen werden. Die Zustandsbestimmung erfolgt in diesen Fällen häufig über mehrfache Umwege, die zeit- und kostenaufwendig sind.

Eine Vorrichtung zur Dichtemessung ist aus der EP-A-0 430 859 bekannt. Diese enthält nur eine an ein Medium aukoppelbare Sendeeinrichtung und nur eine Empfangseinrichtung, wobei die Sendeeinrichtung an einen Eingang eines Komparators und die Empfangseinrichtung an den anderen Eingang des Komparators gelegt ist. Der Ausgang des Komparators ist an eine Verarbeitungseinrichtung gekoppelt.

Es ist Aufgabe der vorliegenden Erfindung, eine Vorrichtung vorzuschlagen, mit der eine Änderung der strukturellen Eigenschaften eines festen, flüssigen oder gasförmigen Mediums mit möglichst geringem Aufwand festgestellt werden kann. Die Vorrichtung soll geeignet sein, auch die strukturellen Eigenschaften von nicht oder schwer zugänglichen Medien in geschlossenen Behältern zu erfassen.

Die Erfindung wird durch die Merkmale des Hauptanspruchs gelöst. Die Unteransprüche betreffen bestimmte Ausführungsformen und Weiterentwicklungen. Die Vorrichtung zur Feststellung der Änderung der Dichte eines Mediums besteht aus einer Sendeeinrichtung zum Aussenden eines beliebigen Sendesignals, wobei das Sendesignal mindestens eine Periode besitzt und die Sendeeinrichtung an das Medium ankoppelbar ist. Weiterhin sind mehrere in einer Fläche oder Zeite angeordnete Empfängseinrichtungen vorhanden, zum Aufnehmen der reflektierten und/oder transmittierten Antwortsignale aus dem Medium, wobei jeder Empfangseinrichtung jeweils ein Komparator folgt, und die Empfangseinrichtung jeweils an den einen Eingang des Komparators und die Sendeeinrichtung jeweils an den anderen Eingang des Komparators gelegt ist. Die Ausgänge der Komparatoren sind mit einer Verarbeitungs- und Auswahleinrichtung gekoppelt, der ein Display folgt.

Eine besonders günstige Ausführungsform der vorliegenden Erfindung entsteht, wenn das Sendesignal eine konstante Frequenz und Amplitudebesitzt.

Diese Vorrichtung zur Feststellung der Änderung der Dichte eines Mediums misst die Phasenverschiebung zwischen dem Sendesignal und dem Empfangssignal, die sich mit Veränderung der Dichte des Mediums ebenfalls verändert. Mit dieser Vorrichtung können also die Änderungen der Dichte eines Mediums verfolgt werden. Zu Beginn der Messung kann bei dieser Vorrichtung bereits eine Phasenverschiebung zwischen dem Sendesignal und dem Empfangssignal vorhanden sein. Die Änderungen der strukturellen Eigenschaften des Mediums führen dann zu einer weiteren Phasenverschiebung, die zur Auswertung kommt.

In einer Weiterentwicklung kann zwischen der Sendeeinrichtung und den Komparatoren jeweils ein einstellbares Verzögerungsglied angeschlossen sein, und die Ausgänge der Komparatoren können mit einer abschaltbaren Rückkopplung verbunden sein, die zu den einstellbaren Verzögerungsgliedern führt. Diese Vorrichtung kann zu Beginn der Messung kalibriert werden, d.h. die Phasenverschiebung zwischen dem Sendesignal und dem Empfangssignal kann zu Beginn der Messung auf Null eingestellt werden. Tritt eine erneute Phasenverschiebung auf, ist diese ausschließlich auf die nach der Kalibrierung eingetretenen Änderungen der Dichte des Mediums zurückzuführen. Wird ein beliebiges Sendesignal verwendet, besteht das Verzögerungslied zum Beispiel aus einem DSP-Prozessor und einem adaptiven Filter. In diesem Fall muss die Phasenverschiebung für jede im Sendesignal enthaltene Frequenz ermittelt werden. Aus diesen Phasenverschiebungen wird dann die Phasenverschiebung des Sendesignals bestimmt.

Sendeeinrichtung und Empfangseinrichtungen müssen bei dieser Vorrichtung stets so zueinander ausgerichtet werden, dass ein möglichst gutes Empfangssignal erhalten wird. Diese Ausrichtung muss während des Messvorganges konstant beibehalten werden. Es ist deshalb vorteilhaft, dass mehrere Empfangseinrichtungen vorhanden sind, die in einer Fläche oder einer Zeile angeordnet sind. Für die Verfolgung der Dichteänderungen wird dann das stärkste Empfangssignal herangezogen.

In anderen Ausführungsformen der vorliegenden Erfindung kann das Sendesignal eine akustische Welle, z.B. Ultraschall darstellen. Diese Vorrichtung ist besonders für medizinische Untersuchungen geeignet. Aber auch in der Metallurgie hat sich der Ultraschall bewährt.

Es ist für unterschiedliche Anwendungsgebiete vorteilhaft, das Sendesignal permanent oder nach einem vorgegebenen Zeitmuster auszusenden. Das Zeitmuster kann dabei auch so gestaltet sein, dass größere Pausen zwischen den Sendesignalen vorhanden sind, weil die Änderungen in der Dichte des Mediums nur sehr langsam verlaufen.

Beim Einsatz der Vorrichtung erfolgt zunächst eine Einstellung des momentanen Zustandes, d.h. der 0-Linie, von der aus die Veränderungen festgestellt werden. Diese "0-Linie" kann durch die im Moment des Anschließens der Vorrichtung an ein Medium vorhandene Phasenverschiebung zwischen dem Sendesignal und dem Empfangssignal gegeben sein, oder durch eine tatsächliche 0-Linie, die durch Kompensation dieser Phasenverschiebung im ersten Messdurchlauf erzeugt wird. Bei dieser sogenannten Kalibrierung ist in der Vorrichtung ein abschaltbares Verzögerungsglied vorhanden. Für die Kalibrierung wird zunächst der von der Sendeeinrichtung erzeugte Sendeimpuls gleichzeitig durch das Medium und das Verzögerungsglied geleitet. Die beiden Impulse, die aus dem Verzögerungsglied bzw. aus der Empfangseinrichtung als Transmissionsimpuls oder Reflexionsimpuls abgenommen werden, werden einem Komparator zugeführt. Wird dabei zwischen den beiden Impulsen eine Phasenverschiebung festgestellt, wird das Verzögerungsglied über die Rückkopplung so eingestellt, dass die Phasenverschiebung eliminiert wird, so dass also zwischen den beiden Impulsen keine Phasenverschiebung mehr vorhanden ist. Damit ist die Vorrichtung kalibriert, d.h. es ist eine tatsächliche "0-Linie" eingestellt, und die Rückkopplung wird abgeschaltet. Da mehrere Empfangseinheiten vorhanden sind, muss die Kalibrierung für jeden Empfangskanal durchgeführt werden. Es kann für die weitere Feststellung von Änderungen der Dichte des Mediums dann der Empfangskanal ausgewählt werden, der die größte Amplitude des Empfangssignals aufnimmt. Ein erneutes Auftreten von Phasenverschiebungen zwischen dem Sendesignal und dem Empfangssignal ist danach nur auf Änderungen der Laufzeit des Sendeimpulses durch das Medium zurückzuführen. Diese Laufzeitänderung kann auch auftreten, wenn ein strömendes flüssiges oder gasförmiges Medium seine Geschwindigkeit ändert. Die größte Amplitude wird dann möglicherweise von einem anderen Empfangskanal aufgenommen. Die einzelnen Empfangseinrichtungen sind dazu flächenhaft oder in einer Zeile einer vorgegebenen Richtung angeordnet. Die Richtung zur Anordnung einer Zeile kann z.B. durch die Strömungsgeschwindigkeit eines flüssigen oder gasförmigen Mediums vorgegeben sein.

Die Art und Weise der Messung entsprechend der vorliegenden Erfindung basiert auf der folgenden Abhängigkeit:

$$T_P = \frac{L}{V}$$

Wobei $T_P$ die Durchlaufzeit des Impulses durch das Medium, V die Ausbreitungsgeschwindigkeit des Impulses durch das Medium und L der Weg zwischen der Sendeeinrichtung und der Empfangseinrichtung darstellt. Ändert sich eine dieser Größen, ändert sich die Durchlaufzeit und damit die Phasenbeziehung der beiden Impulse. Ändert sich die Länge z.B. aufgrund einer Temperatur- oder Druckänderung, wird durch das ΔL auch eine Änderung der Laufzeit $\Delta T_P$ und damit eine Phasenverschiebung eintreten. Eine Änderung der Dichte des Mediums, wie Dichte, Kompressionsmodul führen zur Änderung der Ausbreitungsgeschwindigkeit V und damit ebenfalls zu einem $\Delta T_P$ und einer entsprechenden Phasenverschiebung der beiden Impulse. Aus diesen Beziehungen wird ebenfalls deutlich, dass der absolute Weg zwischen der Sendeeinrichtung und der Empfangseinrichtung nicht mehr in die Messung eingeht, solange er auf dem ursprünglichen Wert verbleibt.

Wenn in einer Anordnung ein gutes Echo erreicht werden kann, sollte die Vorrichtung im Reflexionsverfahren genutzt werden. In den anderen Fällen sollte sie besser im Transmissionsverfahren eingesetzt werden. Eine flächenhafte Anordnung von Einzelempfängern kann das Auffinden des bestens Echos erleichtern.

Mit dieser Vorrichtung ist es möglich, Änderungen in der strukturellen Qualität eines Mediums schnell und einfach zu erfassen. Die Ursache für diese Änderungen kann dabei bekannt sein, muss es aber nicht. Soll ein Stoff eine bestimmte Viskosität besitzen, kann festgestellt werden, wann diese erreicht ist. Füllt sich z.B. ein Gewebe mit Wasser oder Blut oder soll dieses durch therapeutische Maßnahmen aus dem Gewebe entfernt werden, können die Änderungen des Zustandes und die Geschwindigkeit, mit der diese erfolgt, genau erfasst werden.

Ein weites Anwendungsgebiet ergibt sich auch in der chemischen Industrie. Wenn z.B. eine chemische Reaktion überwacht wird, kann der Zeitpunkt des Beginns einer Reaktion oder ihr Abschluss genau bestimmt werden. In diesen Fällen sind lediglich vorher erstellte genaue Messdaten über die Laufzeiten der Sendeimpulse durch die interessierende Sendestrecke im Medium erforderlich.

In Kraftwerken kann mit dieser Vorrichtung z.B. der Zustand des die Turbinen antreibenden Dampfes überwacht werden. Wird an der Rohrwandung der Dampfzuleitung in Richtung der Geschwindigkeit des einströmenden Dampfes eine zeilenförmige Empfangseinrichtung angeordnet, die den transmittierten oder reflektierten Sendeimpuls aufnimmt, kann an den einzelnen Empfangszellen der zeilenförmigen Empfangseinrichtung auch eine Änderung der Geschwindigkeit des Dampfes festgestellt werden. Zur Auswertung der Empfangssignale werden dabei mehrere Empfänger herangezogen.

Die Vorrichtung ist also überall dort mit Vorteil einzusetzen, wo Zustandsänderungen eines Mediums eine positive oder negative Aussage bedeuten, die je nach Art der Änderung oder der Geschwindigkeit, mit der diese eintritt, einen Handlungsbedarf hervorruft. Dabei ist es durchaus möglich, dass die erforderlichen Reaktionen auf die festgestellte Änderung der Dichte des Mediums automatisch eingeleitet werden.

Die Vorrichtung ist einfach und kostengünstig und erfordert keine komplizierten Messabläufe. In der medizinischen Notfallhilfe kann es schnell und sicher Auskunft darüber erteilen, ob z.B. Blut oder eine andere Körperflüssigkeit in das Gehirn oder einen anderen Körperteil einströmt, da dieses zu Änderungen der Dichte des Gewebes führt. Durch weitere Messungen kann dann festgestellt werden, ob die eingeleitete therapeutische Maßnahme oder Notversorgung den gewünschten Erfolg herbeiführt.

Die Erfindung soll nachfolgend anhand eines Ausführungsbeispiels erläutert werden. Gleiche Bezugszahlen bezeichrien in den Zeichnungen gleiche oder ähnliche Teile.

Fig. 1 zeigt ein Beispiel einer nicht-erfindungsgemässen Vorrichtung zur Feststellung von Änderungen der Dichte eines Mediums;

Fig. 2 zeigt ein zweites Beispiel einer nicht-erfindungsgemässen Vorrichtung zur Feststellung von Änderungen der Dichte eines Mediums;

Fig. 3 zeigt ein drittes Beispiel einer nicht-erfindungsgemässen Vorrichtung zur Feststellung von Änderungen der Dichte eines Mediums; und

Fig. 4 zeigt ein Ausführungsbeispiel einer Vorrichtung zur Feststellung von Änderungen der Dichte eines Mediums mit mehreren Empfangskanälen gemäss der vorliegenden Erfindung.

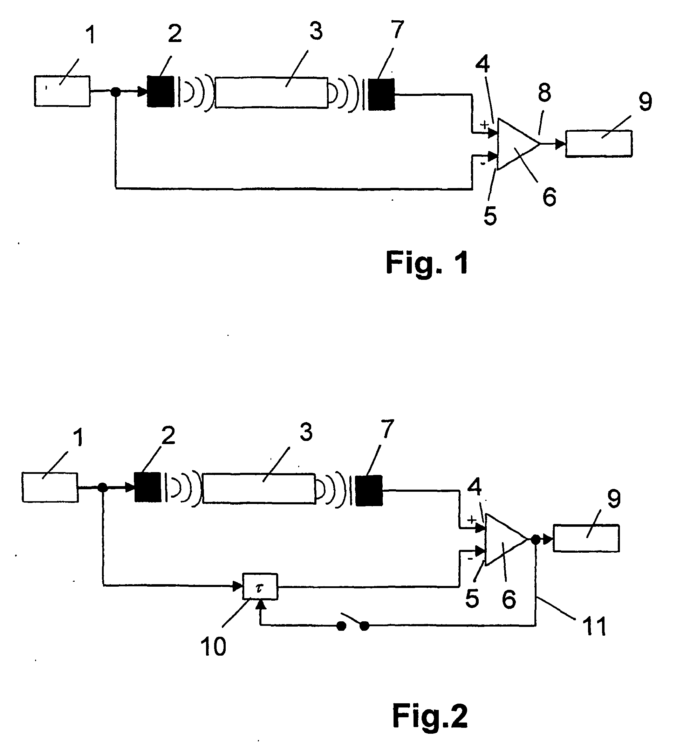

In Fig. 1 ist ein erstes Beispiel einer Vorrichtung zur Feststellung von Änderungen der Dichte eines Mediums in der einfachsten Anordnung dargestellt. Die Vorrichtung zur Feststellung von Änderungen der Dichte eines Mediums 3 besteht aus einem Generator 1 und einer Sendeeinrichtung 2 zum Aussenden eines Sendesignals mit konstanter Frequenz und Amplitude und mindestens einer Periode. Die Sendeeinrichtung 2 ist an das Medium 3 und an einen ersten Eingang 4 eines Komparators 6 gekoppelt. Zum Aufnehmen der in diesem Fall transmittierten Antwortsignale aus dem Medium 3 ist eine Empfangseinrichtung 7 vorhanden, die an den zweiten Eingang 5 des Komparators 6 gekoppelt ist, dessen Ausgang 8 mit einem Display 9 verbunden ist. Im Komparator 6 wird zunächst die Phasenverschiebung zwischen dem Sendesignal und dem Empfangssignal der Empfangseinrichtung 7 ermittelt. Diese Phasenverschiebung wird als Basis genommen. Änderungen der Dichte führen zu Änderungen der ursprünglichen Phasenverschiebung
Fig. 2 zeigt ein zweites Beispiel. Die Vorrichtung zur Feststellung von Änderungen der Dichte eines Mediums ist in diesem Fall dadurch erweitert, dass zwischen der Sendeeinrichtung 2 und dem ersten Eingang 4 des Komparators 6 ein einstellbares Verzögerungsglied 10 angeschlossen ist, so dass das Sendesignal gleichzeitig durch das Medium 3 und das Verzögerungsglied 10 läuft. Außerdem ist der Ausgang 8 des Komparators 6 ist mit einer abschaltbaren Rückkopplung 11 zum einstellbaren Verzögerungsglied 10 verbunden. Mit dieser Anordnung kann die nach dem ersten Sendesignal ermittelte Phasenverschiebung zwischen dem Sendesignal und dem Empfangssignal kompensiert werden. Die Änderungen der Dichte des Mediums 3 werden jetzt auf die Phasenverschiebung Null bezogen. Diese Vorrichtung kann also kalibriert werden.

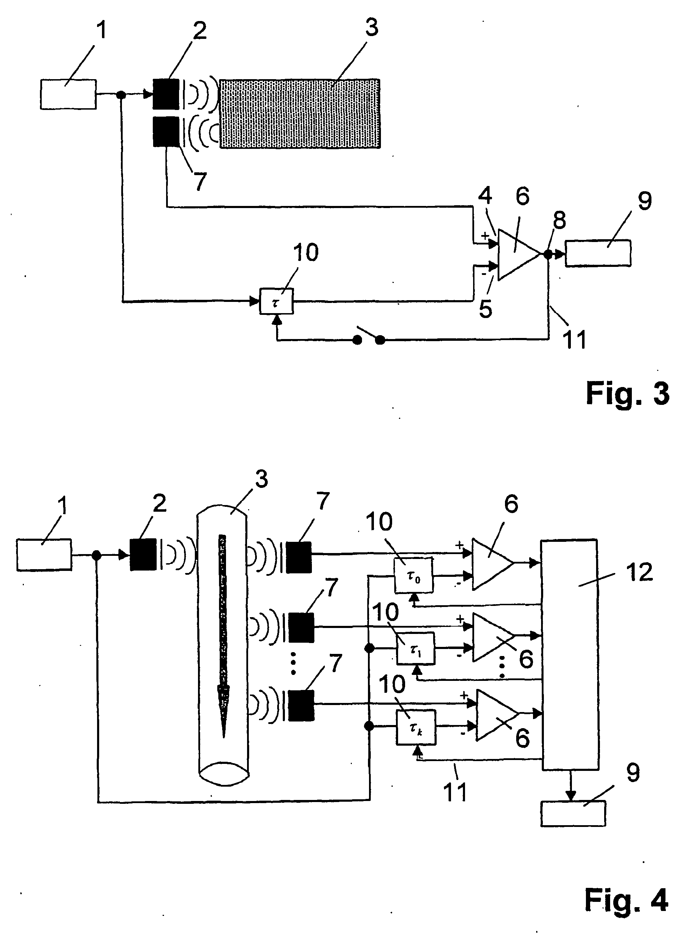

Fig. 3 zeigt ein drittes Beispiel einer Vorrichtung zur Feststellung von Änderungen der Dichte eines Mediums. In der Zeichnung ist ein Generator 1, eine Sendeeinrichtung 2, ein Medium 3, in dem das Sendesignal reflektiert wird, eine Empfangseinrichtung 7 ein Komparator 6, an dessen Eingänge 4 und 5 das Empfangssignal aus der Empfangseinrichtung 7 und das Signal aus dem Verzögerungsglied 10 geführt werden. Der Ausgang 8 des Komparators 6 ist mit einem Display 9 verbunden. Außerdem ist von dem Ausgang 8 des Komparators 6 eine abschaltbare Rückkopplung 11 an das Verzögerungsglied 10 geführt.

In dieser Ausführung kann die Vorrichtung zur Feststellung von Änderungen der Dichte eines Mediums vorteilhaft in der medizinischen Überwachung eines Patienten eingesetzt werden. Es ist bekannt, dass bei einem Hirntrauma eine größere Menge Körperflüssigkeit oder Blut in den Kopf des Patienten eingedrungen sein kann. Mit der Vorrichtung kann z.B. an der Unfallstelle oder dem Ort, an dem ein Patient das Bewusstsein verloren hat, der augenblickliche Zustand festgehalten werden, ohne dass die genaue Ursache bekannt ist. Die Sendeeinrichtung und die Empfangseinrichtung, die aus einem umschaltbaren Sensor bestehen kann, wird dazu an die eine Seite des Kopfes kurz oberhalb des Ohres angelegt, da hier ein gutes Echo des Sendeimpulses an der gegenüberliegenden Schädelwand zu erwarten ist. Liegen Schädelverletzungen vor, muss eventuell ein anderer Ort für den Sensor gewählt werden, so dass ein Reflexionssignal entsteht oder es muss ein Gerät im Transmissionsverfahren verwendet werden. Mit Hilfe des Verzögerungsgliedes 10 kann eine mögliche Phasenverschiebung zwischen dem Sendesignal und dem Empfangssignal durch das Medium 3 kompensiert werden. Danach wird das Verzögerungsglied 10 abgeschaltet. Die Beobachtung der Phasenverschiebung, die danach zwischen dem Sendesignal und dem Empfangssignal durch das Medium 3 auftritt, kann Aufschluss darüber geben, ob sich der Zustand des Patienten verbessert oder verschlechtert. Treten z.B. Blutungen im Gehirn auf, entsteht in diesem Fall eine negative Phasenverschiebung, geht eine mögliche Ansammlung von Körperflüssigkeit zurück, tritt eine positive Phasenverschiebung auf. Die Vorrichtung ermittelt also eine lokale Änderung der Dichte des Gehirns.

In Fig. 4 ist ein Ausführungsbeispiel einer Vorrichtung zur Feststellung von Änderungen der Dichte eines flüssigen oder gasförmigen Mediums entsprechend der vorliegenden Erfindung dargestellt. Die Vorrichtung zur Feststellung von Änderungen der Dichte eines flüssigen oder gasförmigen Mediums 3 besteht aus einem Generator 1 und einer Sendeeinrichtung 2 zum Aussenden eines Sendesignals mit konstanter Frequenz und Amplitude und mindestens einer Periode. Die Empfangseinrichtung 7 besteht in diesem Fall aus mehreren Empfangszellen. Diese Empfangszellen können eine flächenhafte Anordnung besitzen, damit z.B. das stärkste Echo ermittelt und für die Zustandsänderung verwendet werden kann. In dieser Vorrichtung ist die Anzahl der Komparatoren 6, die mit der Empfangseinrichtung 7 verbunden sind, gleich der Anzahl der Empfangszellen in der zeilenförmigen Empfangseinrichtung 7, d.h. die Empfangskanäle bestehen jeweils aus einer Empfangszelle, einem Komparator 6, einem Verzögerungsglied 10 und einer abschaltbaren Rückkopplung 11.

An der gegenüberliegenden Seite des Mediums 3 kann z.B. auch in Strömungsrichtung des flüssigen oder gasförmigen Mediums 3 eine zeilenförmige Empfangseinrichtung 7 angeordnet sein. Die Frequenz des Sendesignals muß dann so gewählt werden, dass das Sendesignal von dem flüssigen oder gasförmigen Medium 3 mitgenommen wird. Das Sendesignal wird je nach Größe der Geschwindigkeit des flüssigen oder gasförmigen Mediums 3 unterschiedlich weit getragen und trifft dann auf den entsprechenden Einzelempfänger der zeilenförmigen Empfangseinrichtung 7 auf. Wobei diese Änderung der Geschwindigkeit nicht explizit ermittelt wird, sondern eine Komponente der Gesamtänderung darstellt. Die Ausgänge 8 der Komparatoren 6 sind mit einer Verarbeitungs- und Auswahleinrichtung 12 verbunden, in der die Phasenverschiebungen zwischen dem Sendesignal und den einzelnen Empfangssignalen ermittelt werden. Es ist dabei möglich, alle Empfangskanäle zu beobachten oder nur einen bestimmten, der nach vorgegebenen Kriterien ausgewählt wurde. Die Verarbeitungs- und Auswahleinrichtung 12 ist mit einem Display 9 verbunden. Wie in dem zweiten und dritten Ausführungsbeispiel bereits erläutert wurde, werden zur Kalibrierung der Vorrichtung in den einzelnen Komparatoren 6 zunächst die Phasenverschiebungen zwischen dem Sendesignal und den einzelnen Empfangssignalen der Empfangseinrichtung 7 ermittelt. Diese Phasenverschiebungen werden dann über die abschaltbaren Rückkopplungen 11 durch die Verzögerungsglieder 10 kompensiert. Änderungen der Dichte führen dann zu Phasenverschiebungen zwischen dem Sendesignal und den einzelnen Empfangssignalen der Empfangseinrichtung 7.

**Patentansprüche**

1. Vorrichtung zur Feststellung der Änderung der Dichte eines Mediums, umfassend eine Sendeeinrichtung (2) zum Aussenden eines beliebigen Sendesignals, wobei das Sendesignal mindestens eine Periode besitzt und die Sendeeinrichtung (2) an das Medium (3) ankoppelbar ist, wobei zum Aufnehmen der reflektierten und/oder transmittierten Antwortsignale aus dem Medium (3) mehrere in einer Fläche oder einer Zeile angeordnete Empfangseinrichtungen (7) vorhanden sind, denen jeweils ein Komparator (6) folgt, wobei die Empfangseinrichtungen (7) jeweils an den einen Eingang der Komparatoren (6) und die Sendeeinrichtung (2) jeweils an den anderen Eingang der Komparatoren (6) gelegt ist, und dass die Ausgänge der Komparatoren (6) mit einer Verarbeitungs- und Auswahleinrichtung (12), die zur Auswahl eines oder aller der von den Empfangseinrichtungen (7) aufgenommenen Antwortsignale und deren weiterer Verarbeitung ausgebildet ist, gekoppelt sind, der ein Display (9) folgt.

2. Vorrichtung zur Feststellung der Änderung der Dichte eines Mediums nach Anspruch 1, bei der das Sendesignal eine konstante Frequenz und Amplitude besitzt.

3. Vorrichtung zur Feststellung der Änderung der Dichte eines Mediums nach Anspruch 1 oder 2, bei der zwischen der Sendeeinrichtung (2) und den Komparatoren (6) jeweils ein einstellbares Verzögerungsglied (10) angeschlossen ist, und bei der die Ausgänge der Komparatoren (6) mit einer abschaltbaren Rückkopplung zu den einstellbaren Verzögerungsgliedern (10) verbunden sind.

4. Vorrichtung zur Feststellung der Änderung der Dichte eines Mediums nach einem der Ansprüche 1 bis 3, bei der das Sendesignal eine akustische Welle darstellt.

5. Vorrichtung zur Feststellung der Änderung der Dichte eines Mediums nach einem der Ansprüche 1 bis 4, bei der das Sendesignal permanent oder nach einem vorgegebenen Zeitmuster aussendbar ist.

**Claims**

1. Device for determining the change in density of a medium, comprising a transmitting device (2) for transmitting any transmission signal, the transmission signal having at least one period and the transmitting device (2) being couplable to the medium (3), wherein for receiving the reflected and/or transmitted response signals from the medium (3) plural receiving devices (7) are present in one area or one line, downstream of which receiving devices (7) a comparator (6) is connected, wherein the receiving devices (7) are each applied to a respective input of the comparators (6) and the transmitting device (2) is applied to the respective other input of the comparators (6), and wherein the outputs of the comparators (6) are coupled to a processing and selection device (12), which is formed for the selection of one or all response signals received by the receiving devices (7) and for their further processing, and are followed by a display (9).

2. Device for determining the change in density of a medium according to claim 1, wherein the transmission signal has a constant frequency and amplitude.

3. Device for determining the change in density of a medium according to claim 1 or 2, wherein between the transmitting device (2) and the comparators (6) a respective adjustable delay member (10) is connected, and wherein the outputs of the comparators (6) are connected to a disconnectable feedback to the adjustable delay members (10).

4. Device for determining the change in density of a medium according to one of claims 1 to 3, wherein the transmission signal has an acoustic wave.

5. Device for determining the change in density of a medium according to one of claims 1 to 4, wherein the transmission signal may be transmitted permanently or according to a specified time pattern.

## Revendications

1. Dispositif destiné à détecter la variation de densité d'un fluide, comportant un émetteur (2) destiné à émettre un signal d'émission quelconque, le signal d'émission ayant au moins une période et l'émetteur (2) pouvant être couplé au fluide (3), dans lequel dispositif plusieurs récepteurs (7), qui sont agencés sur une surface ou sur une ligne, sont prévus pour recevoir les signaux de réponse réfléchis et/ou transmis à partir du fluide (3), un comparateur (6) étant monté en aval de chaque récepteur, chaque récepteur (7) étant raccordé à l'une des entrées des comparateurs (6) et l'émetteur (2) étant raccordé à l'autre entrée des comparateurs (6), et dans lequel dispositif les sorties des comparateurs (6) sont couplées à un dispositif de traitement et sélection (12), qui est conçu pour sélectionner un ou tous les signaux de réponse reçus par les récepteurs (7) et pour effectuer le traitement ultérieur de ces signaux, un écran (9) étant monté en aval dudit dispositif de traitement et sélection.

2. Dispositif destiné à détecter la variation de densité d'un fluide selon la revendication 1, dans lequel le signal d'émission a une fréquence et une amplitude constantes.

3. Dispositif destiné à détecter la variation de densité d'un fluide selon la revendication 1 ou 2, dans lequel un temporisateur (10) réglable est monté entre l'émetteur (2) et chacun des comparateurs (6), et dans lequel les sorties des comparateurs (6) sont reliées à un système de rétroaction, apte à être déconnecté et menant vers les temporisateurs (10) réglables.

4. Dispositif destiné à détecter la variation de densité d'un fluide selon l'une quelconque des revendications 1 à 3, dans lequel le signal d'émission représente une onde sonore.

5. Dispositif destiné à détecter la variation de densité d'un fluide selon l'une quelconque des revendications 1 à 4, dans lequel le signal d'émission peut être émis en permanence ou selon un modèle de temps prédéfini.

**Fig. 1**

**Fig.2**

Fig. 3

Fig. 4